# EUROPEAN PATENT APPLICATION

(11) **EP 1 285 649 A1**
(43) Date of publication of application: **26.02.2003**
(21) Application number: 01810825.8
(22) Date of filing: 23.08.2001
(51) Int. Cl.: A61K 9/24, A61K 31/43, A61K 31/42

(54) **Bilayered dispersible tablet formulation comprising amoxycillin and clavulanate in separate layers**

(71) Applicant: Cimex AG, 4253 Liesberg (CH)
(72) Inventor: Kelbert, Michel, 68730 Blotzheim (FR)
(74) Representative: Arnold, Winfried

(57) **Abstract**

Pharmaceutical formulations for the manufacture of a bilayered dispersible tablet comprising in a first formulation amoxycillin together with pharmaceutically acceptable excipients and in a second formulation clavulanate together with pharmaceutically acceptable excipients, which are used for the manufacture of a bilayered dispersible tablet which is rapidly disintegrating and rapidly releasing the active ingredients into an aqueous fluid, methods for the preparation of such formulations and tablets and their use for oral treatment of bacterial infections.

## Description

### Field of the Invention

This invention relates to novel pharmaceutical formulations comprising amoxycillin and clavulanic acid, which are suitable for the manufacture of oral bilayered, dispersible tablet formulations with rapid disintegrating and rapid dissolution properties. The active ingredients are readily dissolved in aqueous fluids after disintegration of the tablet.

### State of the Art

Amoxycillin is a known beta-lactam antibiotic. Clavulanic acid is a known beta-lactamase inhibitor. Both compounds are used together in a synergistic manner in antibiotic formulations. Clavulanic acid, by inhibiting the enzyme beta-lactamase, serves to prolong the lifetime of amoxycillin in the patient by preventing early destruction thereof. In oral formulations amoxycillin is generally used in the form of its trihydrate, and clavulanic acid is generally used in the form of a pharmaceutically acceptable salt (hereinafter "clavulanate") in particular potassium clavulanate. For example GB 2005538 discloses formulations comprising amoxycillin trihydrate and potassium clavulanate. Such compositions are known and marketed under the trade name Augmentin®.

The Augmentin® tablet on the market is a film tablet. Complete disintegration of this tablet in water of 37° C takes about 10 to 15 minutes.

For certain instances it is of advantage to provide oral pharmaceutical formulations which disintegrate very rapidly in aqueous fluids and, as a second requirement, also rapidly release the active material content in the stomach or intestine.

Sachets contain Augmentin® in form of a powder, which is readily disintegrated in water and immediately forms a dispersion. Sachets have certain drawbacks and in comparison to tablets are expensive to manufacture.

Another powder form is provided in so called "dry syrup" form. The powder is provided in a sufficiently large bottle, to which prior to use the prescribed amount of water is added to give multiple variable doses of a dispersion. The desired amount of the dispersion is applied orally to the patient according to his need. Such multiple variable "dry syrup" is particularly used for children. A drawback of the "dry syrup" powder is that the clavulanate is very sensitive to hydrolysis. After addition of water and upon storage of the dispersion the activity of clavulanate may be diminished or destroyed.

Under other circumstances it is of advantage to release the active ingredient at a controlled, in particular delayed rate. The release of the active material into the stomach or intestine occurs thereby over a period of several hours after ingestion of the formulation. This allows unit doses of the formulation to be taken at widely separated intervals, for example twice daily, whilst maintaining a therapeutically effective plasma level of the active material content.

Achieving a controlled rate of release may be through known layered tablets. GB 1346609 discloses three-layer tablets having two drug containing layers located one on each side of an inert disintegrable intermediate layer. US 4,122,157 discloses a controlled release nitrofurantoin tablet which comprises two adjacent layers, one being a rapid release layer, the other being a slow release layer.

Amoxycillin trihydrate and clavulanate differ substantially with respect to their solubility in water. Potassium clavulanate is freely water-soluble (about 1 part of water for 1 part of solute), whereas amoxycillin trihydrate is only slightly soluble (about 100 to 1000 parts of solvent for 1 part of solute). It is known that clavulanate is relatively sensitive to hydrolysis (maximum stability at a pH of 6.0 to 6.3), so that degradation of clavulanate may occur already in the aqueous fluid or between the relatively long period after oral administration and the contact with the aqueous gastrointestinal fluids.

According to WO 95/20946 a controlled-release tablet formulation for oral administration is disclosed comprising a first layer which includes amoxycillin and/or clavulanate, and a second layer which includes amoxycillin and/or clavulanate, provided that the overall tablet contains amoxycillin, wherein the relative rate of release of amoxycillin and/or clavulanate from the first and second layer differs. For example a first layer is disclosed to be a rapid release layer, which releases the bulk of its active material content within a "relatively short time", for example within 1 hour, e.g. within 30 minutes after oral ingestion. The second slow release layer releases the bulk of its active material content during a relatively long period after oral ingestion or is a delayed release layer which releases the bulk of its active material content after a period of delay after oral ingestion, either in the stomach or in the intestine.

A disadvantage of the bilayered tablet formulation according to WO 95/20946 and other controlled, i.e. slow release formulations is that they are not fast enough dispersible in water which is required for certain purposes. For example the about 1.5 g film coated tablet Augmentan® (German version of Augmentin®⁾ containing 875 mg of amoxycillin and 125 mg of potassium clavulanate, is not dispersible fast enough in water to be orally administered in form of a solution or dispersion. Such tablets cannot be dispersed in water shortly before oral administration. They also will not disperse fast enough in the mucosal fluid.

Oral administration in aqueous solution is in particular desirable for children, elderly or other patients, which cannot swallow large 1.5 g tablets. Such patients include also small animal patients.

In conclusion, the known pharmaceutical formulations comprising amoxycillin and clavulanate have certain disadvantages, drawbacks and limited applicability, which can be summarized as follows:
-- The known film tablets and bilayered tablets do not disintegrate fast enough in water to be useful as dispersible tablets. Some have an unwanted slow release effect.
-- These tablets, in view of their size, may create difficulties in swallowing, especially for children and elderly patients.
-- Conventional homogeneous tablet formulations having a high content of above 875 mg of amoxycillin and 125 mg of clavulanate do not disintegrate fast enough to give a dispersion within 1 to 3 minutes.
-- Sachets contain a powder, which is not intended for direct swallowing, but must be dispersed in water.
-- Only a single dosage is possible. Application of an exact part of a sachet, e. g. of the exact half thereof, is hardly possible for the patient to achieve. Devices are necessary which are usually not available to the patients.
-- Sachets are furthermore expensive to manufacture
-- The powder of "dry syrup" formulations is provided in large bottles. Similar problems exist with respect to administering a defined part of the dispersion.
-- The powder after dispersion in water and storing several days may loose the activity in view of the instability of clavulanic acid.

There is a need for an improved oral pharmaceutical formulation.

### Object of the Invention

It is an object of the present invention to avoid the drawbacks of prior art formulations and to combine their advantages in one pharmaceutical formulation. The present formulations facilitate oral administration. They can be orally administered in various manners. They may be swallowed as a whole, divided in defined smaller parts for application of defined smaller amounts of active ingredients, or rapidly dispersed in an aqueous fluid and administered for example by means of a spoon or drop by drop. Such tablet can be easily used for grown-up patients, children, babies, as well as small and large animals.

It is an object of the present invention to provide a dispersible pharmaceutical tablet formulation for oral administration comprising amoxycillin and clavulanate, which can be orally administered to children and grownup patients without providing any problems in swallowing or other throat problems.

The solution provided by the present invention is a bilayered dispersible tablet, which is readily disintegrated in aqueous fluids, such as water, mucosal or intestinal fluids within up to 1 or 3 minutes. The majority of the particles of the dispersion is preferably smaller than 0.7 mm. The tablets may be divided, swallowed, chewed or dispersed in an aqueous fluid before oral application.

It is an object of the present invention to provide a dispersible bilayered tablet in which the amoxycillin and the clavulanate are separated, each one provided in a separate layer. The tablet is produced from two pharmaceutical formulations comprising the amoxycillin and the clavulanate, respectively, allowing rapid disintegration and rapid simultaneous release of the active principals in aqueous fluids.

In order to provide simultaneous rapid release of the two active ingredients the two formulations must be designed to guarantee rapid disintegration and uniformity of the particles. These goals are achieved by careful selection of the disintegrant, additional ingredients, and other parameters, such as form of the tablet and hardness.

Another object is the easy and commercially advantageous and acceptable preparation of such tablet.

Another object is the use of such tablet in combating infections in a patient in need thereof.

### Detailed Description of the Invention

The objects of the invention are achieved by providing two pharmaceutical formulations of which one comprises an effective amount of amoxycillin and the other an effective amount of clavulanate, both active ingredients along with appropriate pharmaceutically acceptable excipients and coadjuvants, which formulations are suitable for the manufacture of a bilayered dispersible tablet.

### The Pharmaceutical Formulations

The two layers of the bilayered dispersible tablet are formed from two pharmaceutical formulations. The ingredients of the formulations have to be carefully selected to assure rapid disintegration and rapid simultaneous release of the two active ingredients in the aqueous fluid.

Besides amoxycillin and clavulanate the first and second pharmaceutical formulations comprise, independent from each other, additional pharmaceutically acceptable ingredients. These are mainly selected from disintegrants, fillers or diluents, dessicants, lubricants or flow enhancing glidants. Further ingredients may be viscosity enlarging ingredients, tablet binders, colouring agents, and optionally flavoring and/or sweetening agents.

The disintegrant is of particular importance to assure the defined rapid disintegration of the tablet in aqueous fluids. The term "disintegrant" in this description refers to an agent which increases the surface in water so that the tablet is rapidly divided into small particles. Polymers which have a high degree of disintegration power include, inter alia, crosslinked sodium carboxymethylcellulose, cross-linked hydroxypropylcellulose, highmolecular weight hydroxypropylmethylcellulose, carboxymethylamide, potassium methacrylatedivinylbenzene copolymer, polymethylmethacrylate, cross-linked polyvinylpyrrolidone, high-molecular weight polyvinylalcohols, microcrystalline cellulose, and the like. Examples of such polymers include Methocel K4M®, Methocel E5®, Methocel E50®, Methocel E4M®, Methocel K15M® and Methocel K100M®. An example of a suitable polymer mixture is a mixture of Methocel ES® and K4M® in the ratio of 1:1 by weigth. Particular examples of disintegrants are sodium starch glycolate, e. g. Explotab®, polymeric derivatives of acrylic acid, and preferably crosprovidone (an insoluble polyvinylpyrrolidone), e.g. PolyPolyplasdone® XL, or microcrystalline cellulose, e. g. Avicel®.

Sodium starch glycolate may be used in concentrations exceeding 5% by weight in relation to the total weight of the formulation, in particular about 9.5% to 17%.

Polymeric derivatives of acrylic acid may be used in amounts of between about 10% to 21% of the total formulation weight.

Crossprovidone may be used in a concentration of between about 4% and 16%, in particular about 4.15% in the amoxycillin formulation and about 15.5% in the clavulanate formulation of the total formulation weight.

The term "about " as used hereinbefore and hereinafter in connection with weights or other measures may be interpretet to mean plus or minus 10%.

Fillers or diluents facilitate compression of the powder and have an influence on the hardness of the tablet after compression. Such compounds comprise mannitol, microcrystalline cellulose, e. g. Avicel® PH101, in particular cellulose powder, e. g. Elcema® G 250, and silicon dioxide, e.g. Syloid® AL FP.

Fillers are used in amounts of about 5% to 10%, in particular 8.3% in the amoxycillin formulation and about 20% to 50%, in particular 39% in the clavulanate formulation of the total formulation weight.

Lubricants are excipients which reduce inter-particle friction inside the tablet and reduce the reaction forces appearing on the wall of the matrix. Lubricants are for example talcum, stearyl fumarate, polyethyleneglycol, salts of benzoic acid, such as the sodium or lithium salt, and in particular magnesium stearate. Lubricants may be used in amounts of between about 0.1% and 1%, in particular about 0.2% in the amoxycillin formulation and about 0.65% in the clavulanate formulation in relation to the total formulation weight.

Further ingredients may include binders, such as PolyPolyplasdone K29-32®, polyethylene glycol 6000, e. g. Magrocol®^{,} compression aids, and the like.

Flavouring agents have for example the taste of a natural fruit, such as orange, lemon, apple, strawberry, vanilla, or of a herb, for example peppermint, or of broiled or fright meat, such as extracts from liver or yeast.

Sweetening agents are for example, saccharin, aspartam, cyclamate and mixtures thereof. The flavouring and sweetening agents and the amounts should be adjusted to the patient to be treated.

The pharmaceutical formulations of the invention may also include a pH modifying agent, such as a pH buffer, which may be contained in the rapid disintegrating layers. A suitable buffer is calcium hydrogen phosphate.

Colouring agents serve to give a pleasant view and/or to optically distinguish between the two formulations. Such agents are for example tartrazin (E102), chinolin yellow (E104) and yellow orange (E110)

One of the formulations may comprise a colorant agent to easily distinguish one formulation from the other and to provide uniform colour.

Suitably such a rapid release formulation may comprise around 30 - 70 % (all percentages given herein are on a weight percentage basis unless otherwise stated) e.g. 50-60% of active material content, around 10 - 30% of disintegrants, fillers, lubricants, compression aids, viscosity enlarging ingredients, tablet binders, colouring agents, viscosity enlarging ingredients, tablet binders, colouring agents, flavouring and sweetening agents, and the like.

Prefered ingredients in the amoxycillin formulation are in particular crosspovidone, cellulose powder, orange flavour, saccharine, chinolin yellow (E102), and magnesium stearate

Prefered ingredients in the clavulanate formulation are in particular crospovidone, cellulose powder, chinolin yellow (E102) and magnesium stearate

### The Manufacture of the Pharmaceutical Formulations

In a further embodiment the invention provides a method for the manufacture of the two pharmaceutial formulations described hereinabove, characterised in homogeneously mixing amoxycillin with the additional pharmaceutically acceptable excipients and coadjuvants, passing the mixture through a suitable sieve, homogeneously mixing the clavulanate with the additional pharmaceutically acceptable excipients and coadjuvants, and passing the mixture through a suitable sieve. Amoxycillin may be used in the compacted form.

Typically the active material content (may also be amoxycillin powder), disintegrants, fillers, lubricants, viscosity enlarging ingredients, tablet binders, colouring agents, viscosity enlarging ingredients, tablet binders, colouring agents, flavouring and sweetening agents, when used, are mixed, then lubricants and compression aids are added. The complete mixture, in particular when amoxycillin powder is used, may then be compressed under high pressure in the tablet press. Roller compaction may be used to form granulates for tabletting. Alternatively wet granulation may be used, isopropanol being a suitable solvent. A suitable wet granulating aid is Polyplasdone K29-32®.

Clavulanic acid and its derivatives are extremely water sensitive, potassium clavulanate being the most stable pharmaceutically acceptable derivative. Therefore formulations containing derivatives of clavulanic acid, such as potassium clavulanate, should be made up in dry conditions, preferably at 20% relative humidity or less at 20° C, and the ingredients of the formulation should be pre-dried where appropriate. Formulations of the invention including derivatives of clavulanic acid should be stored in containers which are scaled against the ingress of atmospheric moisture.

In particular the active ingredients, amoxycillin powder or clavulanate, fillers and diluents, e. g. cellulose powder, which may contain the colouring agent, or mannitol, release controlling agents (e. g. Methocel®), and disintegrants, e. g. crospovidone (e. g. Polyplestone®) and binders (e. g. Polyplasdone K29-32®) are mixed during 5 minutes in a Stefan® Mixer. Lubricants (e. g. talc, Mg-stearate) and colloidal silicon dioxide (e. g. Syloid ALFP®) are added, and mixing is continued for another minute. The complete mixture is slugged on a Fette® tablet press (briquetting step), followed by size reduction and passaged through an oscillatory 1 mm sieve (Frewitt®). If the flow properties are unsatisfactory, the briquetting step is repeated.

This granulate possesses good flow properties. In some cases, where the bulk density is rather low, a densifying step (pre-tabletting and sieving as in the briquetting method) is still required in order to achieve the nominal weight of a particular layer.

Coating particles and granules with Eudragit ®L is performed in a similar manner. A solution of Eudragit® in isopropanol is mixed with the active gradients or with a granulate (without talc and Mg-stearate) prepared in the aforementioned wet granulation manner. The wet cake is pressed through a 0.5 mm sieve, the mixing is repeated and followed by sieving though a 2 mm sieve.

After drying at 40° C the dry material is passed through a 1 mm sieve. The treatment with Eudragit®, sieving and drying is repeated until the nominal amount of Eudragit L® according to the recipe is contained within the granulate. Then Mg-stearate and talc are added and mixed.

Other coating work with Eudragit L® involved the use of a fluid bed granulator. The active ingredients are spray-coated with an isopropanolic solution of Eudragit L® in a Glatt fluid bed granulator to 20% Eudragit L® of the weight of the active ingredients. Samples are taken at the 5, 10, 15 and 20% Eudragit® weight level. In addition, a granulate of active ingredients with Methocels® is prepared in the Glatt employing an isopropanolic solution of Plasdone K-29-32. This granulate is then spray-coated with Eudragit L® on the Glatt.

### The Use of the Pharmaceutical Formulations

The invention further provides a method of use of a formulation as described above, in the manufacture of a medicament for the treatment of bacterial infections.

The medicament is in particular the bilayered dispersible tablet described herein. The two layers are formed by use of the two pharmaceutical formulations

### The Bilayered Dispersible Tablet

The invention furthermore concerns a bilayered dispersible pharmaceutical tablet for oral administration comprising as pharmaceutically active ingredients in a first layer amoxycillin and in a second layer clavulanate, which is readily dispersible in an aqueous fluid in that both layers are rapidly disintegrating and rapidly and simultaneously releasing the active ingredients in an aqueous fluid.

By the term "bilayered dispersible pharmaceutical tablet" is to be understood a solid preparation containing two single doses of two pharmaceutically active ingredients, each in one of the two layers. The term "dispersible" means the tablet is dispersible within 3 minutes in an aqueous fluid before administration, resulting in a homogeneous dispersion.

Hereinbefore and hereinafter the terms "first" and "second" layer may also be read "one" and "the other" layer, and vice versa.

The bilayered tablet formulation comprises preferably amoxycillin trihydrate and potassium clavulanate in pharmaceutically effective amounts. The overall weight ratio of amoxycillin : clavulanate (expressed as the free acid equivalent) may vary between broad limits, for example between about 30 : 1 to 1 : 1. Suitably the ratio may be between about 8 : 1 to 1 : 1, for example between 7 : 1 to 1 : 1, preferably about 7 : 1 or 8 : 1.

The tablet formulations of the invention may contain up to the maximum permitted daily dose of amoxycillin and clavulanate per unit dose. The formulations may contain for example around 875 mg of amoxycillin and around 125 mg of clavulanate. Suitably unit dose tablets of the invention may contain the following nominal weights (mg) of amoxycillin : clavulanate (expressed as free acid equivalent): ratio 7:1: 875:125; 500:71.428; or 250:35.71; ratio 8:1: 875:109.375; 500:62.5; or 250:31.35: or ratio 4:1: 40:10; 200:50; or 400:100.

Small dose for children, babies or small animals have a weight ratio of amoxycillin : clavulanate from 4 : 1 to 14 : 1, and the weights (mg) may be about 40:10, e .g. also for cats; 200:50, e.g. for small dogs, or 400:100 large dogs.

Large animals, such as horses, cows, pigs and the like need higher dosages, which can be achieved by application of more then one tablet.

In particular the first layer of the present tablet formulation comprises about 875 mg of amoxycillin trihydrate, and the second layer comprises about 125 mg or 109.375 mg of potassium clavulanate.

Preferably bilayered dispersible tablets according to the invention are those described in the Examples. They comprise in the first layer 1027.00 mg of amoxycillin trihydrate, 100.00 mg of cellulose powder (e. g. ELCEMA G 250®) containing about 0.19 mg of chinolin yellow (E104), 50.00 mg of crospovidone (e. g. Polyplasdone XL®), 20.00 mg of orange flavour, 5.00 mg of saccharine and 2.50 mg of magnesium stearate, and in the second layer 149.70 mg of potassium clavulanate, 150.00 mg of cellulose powder (e. g. ELCEMA G 250®) containing about 0.75 mg of chinolin yellow (E104), 60.00 mg of crospovidone (e. g. Polyplasdone XL®), 25. mg of silicium dioxide (e. g. Syloid AL FP®) and 2.50 mg of magnesium stearate,

The hardness of the bilayered dispersible tablet is about 100 to 300 Newton, preferable about 180 Newton.

The form of the tablets is one of the usual round, spherical, block or oblong forms. If intended for swallowing the oblong form is preferred.

### The Manufacture of the Bilayered Dispersible Tablet

In a further embodiment the present invention provides a method for the preparation of a bilayered dispersible tablet, characterised by pressing in a tablet matrix one pharmaceutical formulation of the invention on top of the other.

Suitably the tablet formulations of the invention may be formed by known compression tabletting techniques, for example using a known multi-layer tabletting press. Suitably a dry densification process may be used, e.g. briquetting.

The form of the matrix is the one for achieving the desired form of the tablet.

The manufacture of the bilayered tablets may involve granulation of the pharmaceutical formulation prior to pressing. Preferred is the dry granulation, i. e. without prior wet granulation.

The pressure applied is between about 15 to 50 KN, preferably about 25 KN. The pressure for the first/amoxycillin layer may be lower than the one for the second/clavulanate layer. The resulting hardness is about 100 to 300 Newton, preferable about 180 Newton.

Tablets may have any conventional structure, e. g. by using an oval matrix and a biconvex punch 21 x 10 mm, having a rapid-disintegrating first and second layer.

### The Use of the Bilayered Dispersible Tablet

In a further embodiment the invention provides the use of the bilayered dispersible tablet formulation as an active therapeutic in the treatment of bacterial infections of a host in need thereof.

The bilayered dispersible tablet of the invention may be swallowed, chewed or, before use, dispersed in water or another aqueous fluid of room temperature, e.g. between 15 to 25° C, and administered orally in form of such dispersion. The amount of fluid is not critical. In general an amount for easy swallowing is taken.

Children and babies are conveniently treated with a disintegrated dispersion of a tablet in water, a syrup, or a fruit juice, a tea, or any other pharmaceutically acceptable water containing fluid, eventually spoon by spoon. For animals the tablets may be added to the food, or disintegrated in water and this form added to the food or injected into the mouth by means of a pipette.

For oral administration the amount of the aqueous fluid for one tablet is from e.g. 50 to 100 ml to achieve a concentration of the amoxycillin of about 0.041 % to 2 %, preferably about 0.04 % to 0.875 %, and of clavulanate of about 0.01% to 0.3%, preferably 0.01 % to 0.125 %, +/- 10 %.

The tablet formulations of this invention immediately and simultaneously provide high plasma levels of amoxycillin and clavulanate after oral administration.

The bilayered dispersible tablet is disintegrating in water of room temperature, e.g. of between 15 an 25° C, below 3 minutes, e. g. between about 0.3 and 1 minute, or about 0.5 minutes, and particularly below 1 minute.

Within 5 minutes the bilayered tablet formulation according to the invention releases in water of 37° C about 40 % to 60 %, or an average of about 48.6 %, of the amoxycillin trihydrate , and about 80 % to 95 % or an average of about 78 % of the potassium clavulanate.

Within 30 minutes about 87 % of the amoxycillin trihydrate and about 89 % of the potassium clavulanate is released in water of 37° C.

In a further embodiment the invention provides the use of the bilayered dispersible tablet formulation for preparation of a pharmaceutical package for the treatment of bacterial infections of a host in need thereof. A pharmaceutical package may be any of a suitable package in this field, such as a filled up glass bottle, optionally equipped with a dessicant, or a suitable blister package.

In a further embodiment the invention provides a method of treatment of bacterial infections of a host in need thereof, comprising administering to said host a bilayered dispersible tablet formulation comprising as pharmaceutically acitive ingredients in a first layer amoxycillin and in a second layer clavulanate, characterised in that both layers are rapidly disintegrating and releasing the active ingredients into an aqueous fluid.

The invention also provides a method for the manufacture of a tablet formulation as described above comprising the steps of forming said first and second layers, and any barrier layers and coating layer(s) which may be present.

The invention further provides a method of treatment of bacterial infections in humans or animals which comprises the administration of a therapeutically effective amount of active material content included in a tablet formulation as described herein.

Following is a short description of the accompanying drawings:
Fig 1 shows the percentage dissolution vs. time of amoxycillin and clavulanic acid of six individual tablets prepared according to Example 1 determined in water of 37° C according to the paddle method described in European Pharmacopoeia 1997, 3^{rd} Ed., page 128.
Fig. 2 shows the percentage dissolution vs. time of amoxycillin of six individual tablets prepared according to Example 1 in water of 37° C using the paddle method as described under Fig. 1
Fig. 3. shows the percentage dissolution vs. time of potassium clavulanate of six individual tablets prepared according to Example 1 in water of 37° C using the paddle method as described under Fig. 1
The following examples serve to further characterise the invention, however, should not be construed as a limitation thereof.

### Example 1. Preparation of bilayered dispersible tablets with 1027 mg of amoxicillin trihydrate and 149.7 mg of potassium clavulanate (7:1)

The ingredients for the two formulations of one tablet are given in the following Tables I and II.

**Table 1,**

| **I. Amoxycillin containing formulation for one tablet layer** | | |
|---|---|---|
| | **Compounds** | **mg/Tablet** |
| 1 | amoxycillin trihydrate, compacted | 1027.00 |
| 2 | cellulose powder (ELCEMA G 250®), containing 0.19 mg of chinolin yellow (E104) | 100.19 |
| 3 | crospovidone (Polyplasdone XL®) | 50.00 |
| 4 | orange flavour | 20.00 |
| 5 | saccharine | 5.00 |
| 6 | magnesium stearate | 2.50 |
| **Total weight of the amoxycillin formulation** | | **1204.69** |

The ingredients 1 - 5 of the amoxycillin formulation I are passed through a 1.0 mm oscillatory sieve and for 10 minutes at 15 rpm carefully mixed. The lubricant ingredient 6 is added and mixing continued for 3 minutes at 15 rpm.

**Table 2,**

| **II. Clavulanate containing formulation for the other tablet layer** | | |
|---|---|---|
| | **Compounds** | **mg/Tablet** |
| 7 | potassium clavulanate | 149.70 |
| 8 | cellulose powder (ELCEMA G 250®) containing 0.75 mg of chinolin yellow (E104) | 150.75 |
| 9 | crospovidone (Polypiasdone XL®) | 60.00 |
| 10 | silicon dioxide (Syloid AL FP) | 25.00 |
| 11 | magnesium stearate | 2.50 |
| **Total weight of clavulanate formulation** | | **387.95** |

The ingriedients 7 - 10 of the clavulanate formulation II are passed through a 1.0 mm oscillatoy sieve and carefully mixed for 10 minutes at 15 rpm . The lubricant ingredient 11 is added and mixing continued for 3 minutes at 15 rpm.

The complete mixture is slugged on a rotary tablet press or alternatively compacted with a Bepex® compactor at a pressure of about 45 KN, followed by size reduction with an oscillatory 1.0 mm sieve (Frewitt®). The mixture is kept at below 20% room humidity at 20° C

### Preparation of the bilayered dispersible tablet

The amoxycillin formulation I is first filled into an oblong tablet matrix of 20.7 × 10.8 mm and pressed using an oblong punch with 1204.69 mg. The clavulanat formulation II is added and pressed with 387.95 mg. A bilayerd dispersible tablet with a total weight of 1592.64 mg results.

For the production of 300 000 tablets aliquots of the numbers in grams are used.

The tablet is 8.8 mm high. and easily dividable, in particular when one or two break marks are present. The hardness is about 150 - 200 N. The disintegrating time is below 1 minute in water at 20° C.

### Example 2. Disintegration Report

Six randomly selected tablets of Example 1 were tested in the basket apparatus at 20° C according to European Pharmacopeia, 1997, 3^{rd}. Edition, page 127, and compared in a parallel test with randomly selected six commercially available coated Augmentan® (Beecham Germany) tablets containing 1004.31 mg of Amoxycillin trihydrate and 148.83 mg of potassium clavulanate. The results are shown in Table 3.

**Table 3**

| **Disintegrating time in minutes at 20° C** | |
|---|---|
| Augmentan® | Tablets of Example 1 |
| 14.2 | 0.38 |
| 14.4 | 0.4 |
| 15.1 | 0.4 |
| 15.4 | 0.44 |
| 15.5 | 0,53 |
| 16.5 | 1.0 |

The comparative test results indicate that the disintegrating time of Augmentan® is too high for use as a dispersible tablet, whereas the disintegrating time for the tablet according to the invention fulfills the requirements for a dispersible tablet.

### Example 3. Dissolution Report

Six randomly selected bilayered dispersible tablets of Example 1 were tested in a paddle apparatus according to European Pharmacopoeia 1997, page 128. One tablet was put into 900 ml of water of 37° C. Stirring was performed with 75 rpm. The content of active ingredient in the water was determined by HPLC analysis immediately, and after 5, 10, 15 and 30 minutes. The results are compiled in the following tables.

**Table 4:**

| **Amoxycillin** | | | | | | | | |
|---|---|---|---|---|---|---|---|---|
| **Minutes** | **Tablet No. - % dissolved** | | | | | | **Average** | **Srel** |
| | **1** | **2** | **3** | **4** | **5** | **6** | | |
| 0 | 0.00 | 0.00 | 0.00 | 0.00 | 0.00 | 0.00 | 0.00 | 0.00 |
| 5 | 45.28 | 51.22 | 50.46 | 53.62 | 47.69 | 43.50 | 48.63 | 7.88 |
| 10 | 57.88 | 68.29 | 61.89 | 58.06 | 64.47 | 57.18 | 61.30 | 7.24 |
| 15 | 67.50 | 79.40 | 70.28 | 67.62 | 74.69 | 66.68 | 71.03 | 7.10 |
| 30 | 83.37 | 95.38 | 83.25 | 84.96 | 91.46 | 83.79 | 87.04 | 5.90 |

**Table 5:**

| **Clavulanate** | | | | | | | | |
|---|---|---|---|---|---|---|---|---|
| **Minutes** | **Tablet No. - % dissolved** | | | | | | **Average** | **Srel** |
| | **1** | **2** | **3** | **4** | **5** | **6** | | |
| 0 | 0.00 | 0.00 | 0.00 | 0.00 | 0.00 | 0.00 | 0.00 | 0.00 |
| 5 | 94.43 | 59.91 | 90.90 | 80.85 | 58.51 | 84.47 | 78.18 | 19.76 |
| 10 | 94.05 | 67.22 | 90.65 | 81.74 | 64.79 | 84.88 | 80.55 | 15.01 |
| 15 | 94.32 | 74.42 | 90.54 | 83.33 | 70.24 | 85.78 | 83.11 | 11.15 |
| 30 | 94.22 | 88.81 | 90.44 | 87.09 | 85.83 | 89.13 | 89.25 | 3.27 |
| Srel = relative standard deviation | | | | | | | | |

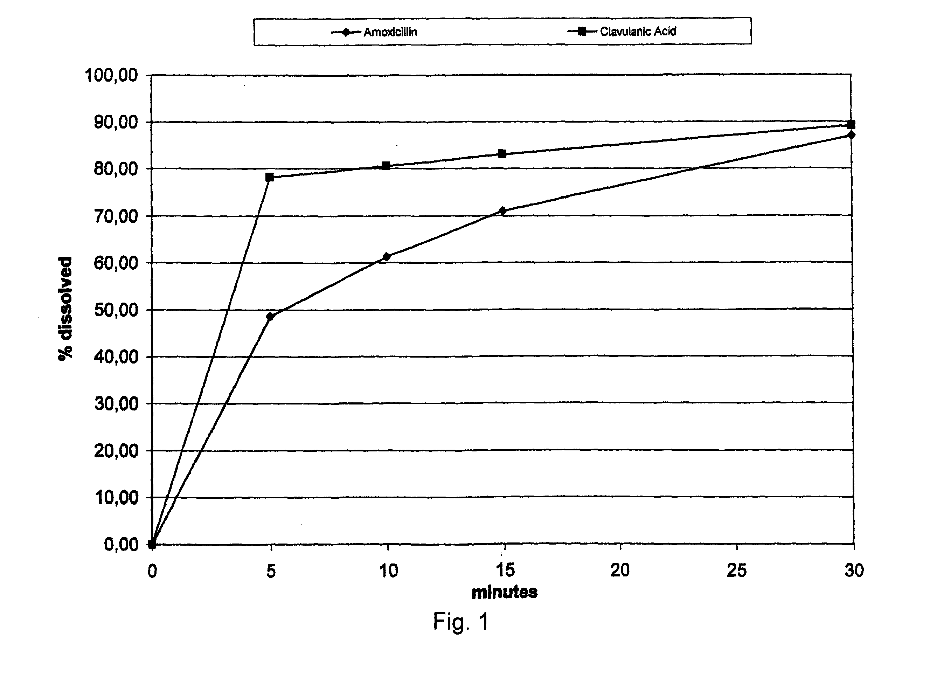

The results of the tables 4 and 5 are graphically shown in Figures 1, 2, and 3, respectively.
Fig. 1 shows the average dissolution of six tablets of amoxycillin and clavulanate of Table 4 and 5 from 0 to 30 minutes .

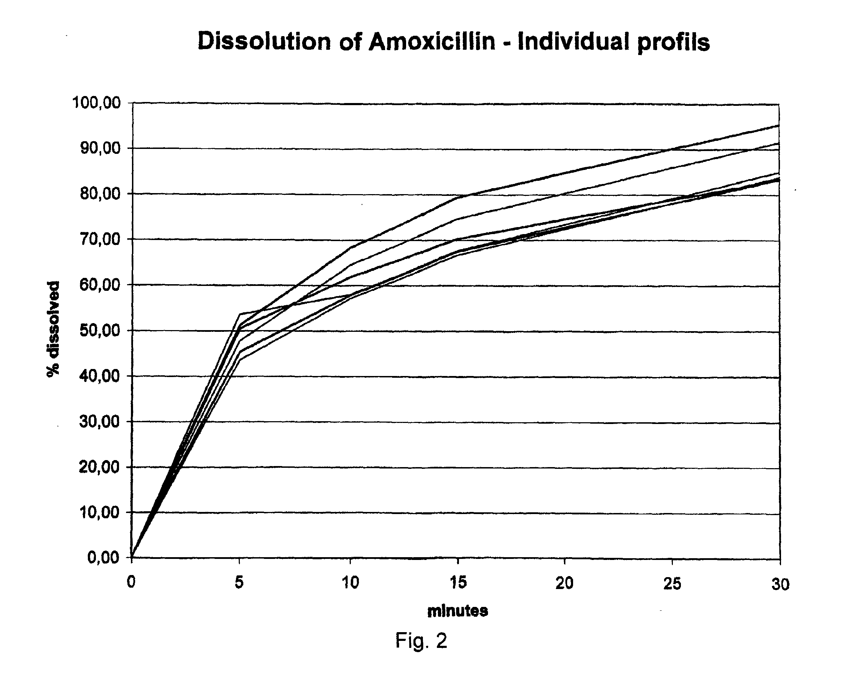

Fig. 2 shows the individual dissolution profiles of the six tablets of Table 4 with respect to amoxycillin.

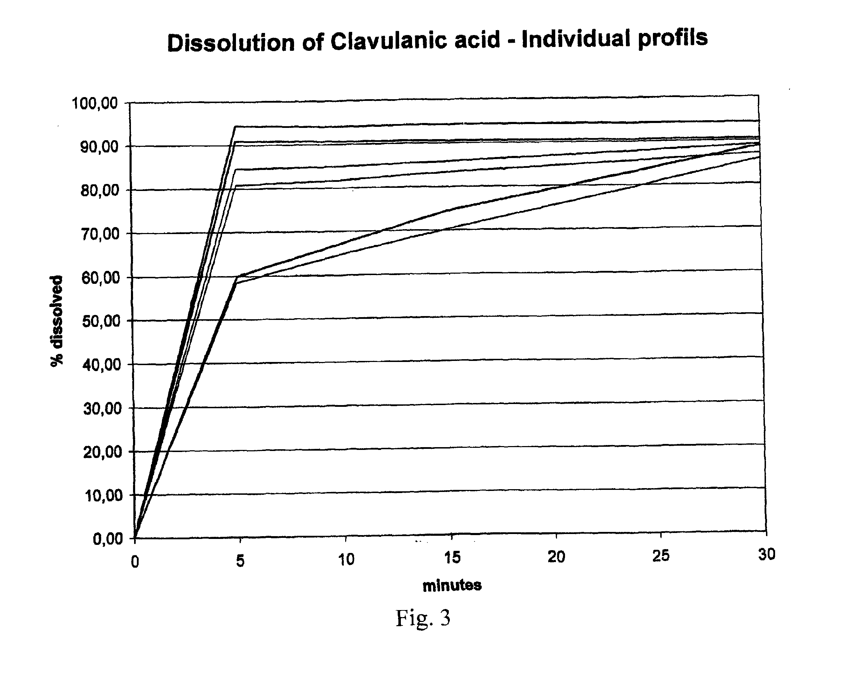

Fig. 3 shows the individual dissolution profiles of the six tablets of Table 5 with respect to clavulanate.

### Example 4. Preparation of bilayered dispersible tablets with 1027 mg of amoxicillin trihydrate and 130.89 mg of potassium clavulanate (8:1)

The ingredients of the two formulations are given in Tables 6 and 7.

**Table 6,**

| **I. Amoxycillin containing formulation for one tablet layer** | | |
|---|---|---|
| | **Compounds** | **mg/Tablet** |
| 1 | amoxycillin trihydrate, compacted | 1027.00 |
| 2 | cellulose powder (ELCEMA G 250®), containing 0.19 mg of chinolin yellow (E104) | 100.19 |
| 3 | crospovidone (Polyplasdone XL®) | 50.00 |
| 4 | orange flavour | 20.00 |
| 5 | saccharine | 5.00 |
| 6 | magnesium stearate | 2.50 |
| **Total weight of the amoxycillin formulation** | | **1204.69** |

**Table 6,**

| **II. Clavulanate containing formulation for the other tablet layer** | | |
|---|---|---|
| | **Compounds** | **mg/Tablet** |
| 7 | potassium clavulanate | 130.98 |
| 8 | cellulose powder (ELCEMA G 250®) containing 0.75 mg of chinolin yellow (E104) | 131.25 |
| 9 | crospovidone ( Polyplasdone XL®) | 52.50 |
| 10 | silicon dioxide (Syloid AL FP) | 21.875 |
| 11 | magnesium stearate | 2.18 |
| **Total weight of clavulanate formulation** | | **339.44** |

The manufacture of the two formulations and the final tablet is the same as described in Example1. The final tablet has a weight of 1544.13 mg.

Similar results are obtained for disintegration and dissolution as in Example 2 and 3.

## Claims

1. A first pharmaceutical formulation comprising a therapeutically effective amount of amoxycillin and a second pharmaceutical formulation comprising a therapeutically effective amount of clavulanate, both active ingredients along with appropriate pharmaceutically acceptable excipients and coadjuvants including a disintegrant, which formulations are suitable for the manufacture of a bilayered dispersible tablet.

2. A pharmaceutical formulation according to claim 1, wherein said appropriate excipients and coadjuvants comprise also dessicants, lubricants, sweeteners and flavourings.

3. A pharmaceutical formulation according to claims 1 or 2, wherein amoxycillin is present in form of the trihydrate.

4. A pharmaceutical formulation according to claims 1 or 2, wherein clavulanate is present in form of potassium clavulanate.

5. A first and a second pharmaceutical formulation according to claims 1-4, wherein in combination the ratio of amoxycillin trihydrate and potassium clavulanate is between about 4 : 1 to 14 : 1.

6. A first and a second pharmaceutical formulation according to claims 1-5, wherein in combination the ratio of amoxycillin trihydrate and potassium clavulanate is about 7 : 1 or 8 : 1.

7. A first and a second pharmaceutical formulation according to claims 1-6, wherein the first comprises about 875 mg of amoxycillin trihydrate and the second about 125 mg of potassium clavulanate.

8. A first and a second pharmaceutical formulation according to claims 1-6, wherein the first comprises about 875 mg of amoxycillin trihydrate and the second about 109.375 mg of potassium clavulanate.

9. A first and a second pharmaceutical formulation according to claims 1-8, comprising additional pharmaceutically acceptable ingredients, selected from disintegrants, viscosity enlarging ingredients, fillers, dessicants, lubricants, tablet binders and optionally colouring, flavoring and/or sweetening agents.

10. A first and a second pharmaceutical formulation according to claims 1-9, comprising additional pharmaceutically acceptable ingredients, selected from crospovidone, cellulose powder, silicon dioxide and magnesium stearate, and optionally, colouring, flavoring and sweetening agents.

11. A first pharmaceutical formulations according to claims 1-10, comprising 1027.00 mg of amoxycillin trihydrate, 100.00 mg of cellulose powder (e. g. ELCEMA G 250®) with admixed 0.19 mg of chinolin yellow, 50.00 mg of crospovidone (e. g. Polyplasdone XL®), 20.00 mg of orange flavor, 5.00 mg of saccharine and 2.50 mg of magnesium stearate.

12. A second pharmaceutical formulation according to claims 1-10, comprising 149.70 mg of potassium clavulanate, 150.00 mg of cellulose powder (e. g. ELCEMA G 250®) with admixed 0.75 mg of chinolin yellow, 60.00 mg of crospovidone (e. g. Polyplasdone XL®), 25 mg of silicon dioxide (e. g. Syloid ALFP®) and 2.50 mg of magnesium stearate,

13. A first and a second pharmaceutical formulation according to claims 1-12, of which the tablet manufactured thereof is disintegrating in aqueous fluids of room temperature within about 0.4-1 minute.

14. A first and a second pharmaceutical formulation according to claims 1-13, of which the tablet manufactured thereof is disintegrating in aqueous fluids of room temperature within 1 minute.

15. A first and a second pharmaceutical formulation according to claims 1-14, of which the tablet manufactured thereof is disintegrated in an aqueous beverage selected from water, fruit juice, syrup, and the like, and administered orally.

16. A first and a second pharmaceutical formulation according to claims 1-15,. of which the tablet manufactured thereof is disintegrated in water and about 50 % of the amoxycillin trihydrate is dissolved in water of 37°C within 5 minutes.

17. A first and a second pharmaceutical formulation according to claims 1-16, of which the tablet manufactured thereof is disintegrated in water and about 87% of the amoxycillin trihydrate is dissolved in water of 37°C within 30 minutes.

18. A first and a second pharmaceutical formulation according to claims 1-17, of which the tablet manufactured thereof is disintegrated in water and about 78 % of the potassium clavulanate is dissolved in water of 37°C within about 5 minutes.

19. A first and a second pharmaceutical formulation according to claims 1-18, of which the tablet manufactured thereof is disintegrated in water and about 89% of the potassium clavulanate is dissolved in water of 37°C within about 30 minutes.

20. A method for the manufacture of the two pharmaceutial formulations as set forth in claims 1-19, **characterised in** homogeneously mixing amoxycillin with the additional pharmaceutically acceptable excipients and coadjuvants, passing the mixture through a suitable sieve, homogeneously mixing the clavulanate with the additional pharmaceutically acceptable excipients and coadjuvants, passing the mixture through a suitable sieve

21. A method of use of a formulation as set forth in any one of claims 1-19, for the manufacture of a medicament for the treatment of bacterial infections.

22. A method of use according to claim 21, wherein the medicament is the bilayered dispersible tablet.

23. A bilayered dispersible pharmaceutical tablet for oral administration comprising as pharmaceutically acitive ingredients in a first layer amoxycillin and in a second layer clavulanate, which is readily dispersible in an aqueous fluid in that both layers are rapidly disintegrating and rapidly releasing the active ingredients into an aqueous fluid.

24. A bilayered dispersible pharmaceutical tablet according to claim 23, comprising a first and a second pharmaceutical formulation as set forth in anyone of claims 1-19.

25. A bilayered dispersible pharmaceutical tablet according to claim 23-24, having a hardness of about 100 to 300 Newton, preferable about 180 Newton.

26. A method for the manufacture of a bilayered dispersible tablet, **characterised by** pressing in a tablet matrix one pharmaceutical formulation as set forth in anyone of Claims 1-19 on top of the other.

27. A method for the manufacture of a bilayered, dispersible tablet comprising the steps of forming a first and second layer by means of a first and a second pharmaceutical formulation according to any one of claims 1-19.

28. Use of the bilayered dispersible tablet formulation as set forth in claim1 23-25 as an active therapeutic in the treatment of bacterial infections of a host in need thereof.

29. A method of treatment of bacterial infections of a host in need thereof, comprising administering to said host a pharmaceutical tablet according to claims 23-25.
